Europäisches Patentamt

European Patent Office

Office européen des brevets

(19)

(11) Veröffentlichungsnummer: **0 168 740**
**A1**

(12) **EUROPÄISCHE PATENTANMELDUNG**

(21) Anmeldenummer: 85108425.1

(22) Anmeldetag: 08.07.85

(51) Int. Cl.⁴: **A 61 F 5/56**

(30) Priorität: **14.07.84 DE 3426095**

(43) Veröffentlichungstag der Anmeldung: **22.01.86**
**Patentblatt 86/4**

(84) Benannte Vertragsstaaten: **AT BE CH DE FR GB IT LI LU NL SE**

(71) Anmelder: **Panic, Milan, Gelsenkirchener Strasse 2, D-5000 Köln 60 (DE)**

(72) Erfinder: **Panic, Milan, Gelsenkirchener Strasse 2, D-5000 Köln 60 (DE)**

(74) Vertreter: **Baur, Eduard, Dr.-Ing. Dipl.-Ing., Werderstrasse 3, D-5000 Köln 1 (DE)**

(54) **Verfahren und Vorrichtung zum Unterdrücken des Schnarchens.**

(57) Zum Unterdrücken des Schnarchens, bei dem bei einem Auftreten des Schnarchens in einem Sender ein Gegenton erzeugt wird, wird vorgeschlagen, daß der Gegenton aus einer raschen Folge von Schnalztönen besteht, wobei vorteilhaft in einem Zeitraum von fünf Sekunden 15 bis 20 Schnalztöne erfolgen.

-1-

Patentanmeldung
================================

des Herrn Milan Panič, Gelsenkirchener Str. 2, 5000 KÖLN 60


Verfahren und Vorrichtung zum Unterdrücken des Schnarchens.


Die Erfindung betrifft ein Verfahren und eine Vorrichtung zum Unterdrücken des Schnarchens.


Das Schlafen von mehreren Personen in einem Raum, darunter fällt auch
das Schlafen von Ehepaaren in einem Bett oder in zwei nebeneinander
stehenden Betten, ist es vielfach unangenehm, sofern ein Partner schnarcht.
Dieses Schnarchen wird vielfach auch mit "Sägen" bezeichnet. Um dieses
Schnarchen zu vermeiden, sind vielfache Vorschläge gemacht worden. So
geht ein Vorschlag dahin, bei dem Auftreten eines Schnarchtones diesen
zu erfassen und damit ein Gerät mit leichter Musik einzuschalten. Das
Ertönen einer leichten Musik hat aber nur bei wenigen Menschen die
Wirkung, daß sie das Schnarchen einstellen. Das Ertönen einer Musik
führt oft zum Aufwecken, stört den Schlaf und verhindert in vielen
Fällen einen Tiefschlaf.


Ein anderer Vorschlag geht dahin, am Kopfende des Bettes eine Alarmschiene zu befestigen und in Verbindung mit einem Draht unter jedem
Kopfkissen ein Mini-Mikrophon zu legen, dieses Mini-Mikrophon hört
dann, welcher Schläfer schnarcht und beginnt ganz leise zu piepen.

0168740

Die Piep-Töne veranlassen den Schnarcher, sich umzudrehen. Mit der Umdrehbewegung hört er dann auf zu schnarchen. Nachteilig bei dieser Maßnahme ist, daß der Schnarcher nach sehr kurzer Zeit wieder zu Schnarchen  beginnt mit dem Ergebnis, daß das Mini-Mikrophon den erneuten Schnarchton empfängt und einen zusammenhängenden Piepton oder mehrere einzelne Pieptöne aussendet und den Schnarchenden veranlaßt, sich wiederum umzudrehen, wobei dann eine kurze Zeit das Schnarchen aufhört, bis es wieder erneut einsetzt. Auch das mehr oder weniger langanhaltende Piepen in Verbindung mit einem ständigen Hin- und Herdrehen führt zu einem unruhigen Schlaf. Dazu kommt die Gefahr, daß der Schlafende sich in dem Draht verfangen kann.

Die vorliegende Erfindung geht von der Aufgabe aus, ein Verfahren und eine Vorrichtung zum Unterdrücken des Schnarchens zu schaffen, bei dem mit einfachen Mitteln das Schnarchen ohne ein Umdrehen des Schlafenden beendet und ein durchgehend tiefer Schlaf erreicht wird.

Zur Lösung dieser Aufgabe wird bei einem Verfahren zum Unterdrücken des Schnarchens, bei dem bei einem Auftreten des Schnarchens ein Gegenton erzeugt wird, erfindungsgemäß vorgeschlagen, daß der Gegenton aus einer raschen Folge von Schnalztönen besteht.

Es wurde überraschend festgestellt, daß Schnalztöne das Schnarchen verhindern und einen Schnarchenden veranlassen, das Schnarchen einzustellen. Dabei haben Schnalztöne die Eigenschaft, daß sie weder die zunächst schnarchende Person noch sonstige Personen wecken und nicht lediglich das Schnarchen verhindern, solange sie ertönen, sondern auch auf den Schlafenden und dessen Unterbewußtsein sehr beruhigend einwirken, so daß die Neigung oder Veranlagung zum Schnarchen durch Schnalztöne stark vermindert und der Tiefschlaf begünstigt wird.

Die Einwirkung von Schnalztönen auf einen Schlafenden, der die Neigung zum Schnarchen hat oder vielfach schnarcht, ist so beruhigend, daß in rascher Folge auftretende Schnalztöne sehr leise sein können, weil sie im Unterbewußtsein des Schlafenden aufgenommen werden. Dies führt dazu, daß es vielfach zweckmäßig ist, die Schnalztöne nicht erst dann ertönen zu lassen, sofern der Schläfer schnarcht, sondern die Schnalztöne bei einer zum Schnarchen neigenden Person in ganz geringer Lautstärke ständig oder zu Zeiten, in denen das Schnarchen besonders zu befürchten ist, z.B. bei einigen Personen nach Alkoholgenuß, ertönen können, weil sie das Schlafen nicht behindern, sondern sogar begünstigen.

Die Schnalztöne werden dadurch erzeugt, daß die Zungenspitze an den Oberkiefer im Bereich der Mitte der zugeordneten Zahnreihe fest bzw. stark angedrückt und dann ganz schnell nach unten frei gelassen wird. Dies geschieht mit einer solch schnellen Folge, daß in einem Zeitraum von fünf Sekunden 15 bis 20 Schnalztöne erfolgen. Dies bedeutet, daß in einem Zeitraum von einer Sekunde etwa 2 bis 5, vorzugsweise 3 bis 4 Schnalztöne erfolgen.

Diese Angabe der Frequenz, in der die Schnalztöne erfolgen, soll nicht besagen, daß die Schnalztöne lediglich nur über einen Zeitraum von fünf Sekunden erfolgen sollen. Vielmehr können diese nach den zwei Arten erfolgen:

a) die Schnalztöne in vorgenannter Frequenz erfolgen über den Zeitraum, in dem der Schlafende tatsächlich schnarcht,

b) die Schnalztöne erfolgen über einen längeren Zeitraum, um das Schnarchen nicht erst aufkommen zu lassen.

Eine Vorrichtung zur Durchführung der vorgenannten Verfahren besteht besonders vorteilhaft aus einem Empfänger und einem Sender, wobei der Sender in rascher Folge die Schnalztöne ausstrahlt. Dieser Sender und Empfänger wird in einem geringen Abstand am Kopfende eines Bettes angebracht. Er kann an einer Wand befestigt sein. Er kann aber auch mit einer Nachttischlampe oder Teil des Schlafzimmers integriert sein, so daß er unauffällig ist.

Besonders vorteilhaft weist der Sender einen Tonträger auf, auf dem die Schnalztöne gespeichert sind, die in schneller Folge in angegebener Frequenz wiedergegeben werden. Da die Schnalztöne eine gleiche Tonhöhe bzw. Tonlage haben, ist der Tonträger von nur geringer Bemessung. So genügt bei einem umlaufenden Tonträger die Anordnung einer einzigen Tonrinne. Der Tonträger kann die verschiedenartigsten Raumformen haben.

Die Erfindung ist in der Zeichnung beispielhaft dargestellt.

Es zeigen:

Figur 1          einen vertikalen Schnitt durch die Vorrichtung,

Figur 2          den Tonträger in Oberansicht.

Die Vorrichtung besteht aus einem Gehäuse 10, an dem der Empfänger 11
angeordnet ist. Dieser betätigt einen kleinen Elektromotor 12 mit
einem Tonträger 13. Vorhanden ist die Tonabnahme 14, die mit einem
Lautsprecher 15 als Sender verbunden ist, der die auf den Tonträger 13
gespeicherten Schnalz-Töne ausstrahlt.

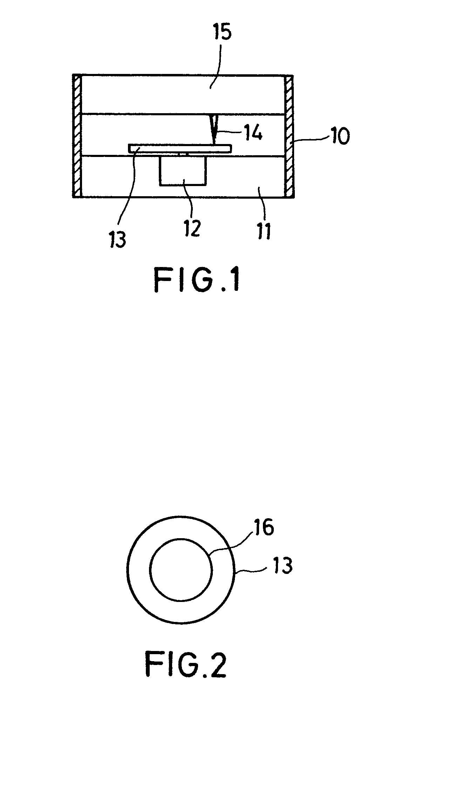

Figur 2 zeigt den Tonträger 13, der nur eine einzige Tonrinne 16 hat.
Der Tonträger kann verschiedenartigste Raumformen haben, so auch ein
Magnettonband sein.

Der Abstand des Lautsprechers zum Kopf des Schlafenden beträgt
vorzugsweise 1 bis 2 m.

Die Wiedergabevorrichtung ist vorzugsweise von elektronischer Art.

- Patentansprüche -

Patentansprüche 0168740

1. Verfahren zum Unterdrücken des Schnarchens, bei dem bei einem Auftreten des Schnarchens ein Gegenton erzeugt wird, d a d u r c h  g e k e n n z e i c h n e t ,  daß der Gegenton aus einer raschen Folge von Schnalz-Tönen besteht.

2. Verfahren nach Anspruch 1,  d a d u r c h  g e k e n n - z e i c h n e t ,  daß in einem Zeitraum von fünf Sekunden 15 bis 20 Schnalz-Töne erfolgen.

3. Vorrichtung zur Durchführung der Verfahren nach den Ansprüchen 1 und 2,  g e k e n n z e i c h n e t  d u r c h  einen Empfänger (11) und einen Sender (15), wobei der Sender in Rascher Folge Schnalz-Töne ausstrahlt.

4. Vorrichtung nach Anspruch 3,  d a d u r c h  g e k e n n - z e i c h n e t ,  daß der Sender einen Tonträger (13) aufweist, auf dem die Schnalztöne gespeichert sind.

5. Vorrichtung nach Anspruch 3,  d a d u r c h  g e k e n n - z e i c h n e t ,  daß der Tonträger (13) nur eine einzige Tonrinne (16) aufweist.

6. Vorrichtung nach Anspruch 3,  d a d u r c h  g e k e n n - z e i c h n e t , daß sie von elektronischer Art ist.

0168740

FIG.1

FIG.2

Europäisches
Patentamt

**EUROPÄISCHER RECHERCHENBERICHT**

0168740
Nummer der Anmeldung

EP 85 10 8425

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| A | US-A-3 696 377 (WALL)<br>* Ansprüche 1,2,4; Spalte 1, Zeilen 42-46 *<br>--- | 1,3,4 | A 61 F 5/56 |
| A | DE-A-1 810 567 (SCHAIBLE)<br>* Ansprüche 1,2; Seite 2, Zeilen 7-21 *<br>--- | 3 | |
| A | FR-A-1 291 387 (ROSSET)<br>--- | | |
| A | DE-A-2 365 025 (SCHREP)<br>--- | | RECHERCHIERTE SACHGEBIETE (Int. Cl.4) |
| A | DE-U-8 105 822 (WENTKER)<br>----- | | A 61 F 5/00 |

Der vorliegende Recherchenbericht wurde für alle Patentansprüche erstellt.

| Recherchenort<br>BERLIN | Abschlußdatum der Recherche<br>08-10-1985 | Prüfer<br>KANAL P K |
|---|---|---|

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82